Europäisches Patentamt

European Patent Office

Office européen des brevets

(11) Numéro de publication: **0 292 402 B1**

# (12) FASCICULE DE BREVET EUROPEEN

(45) Date de publication de fascicule du brevet: **18.11.93**    (51) Int. Cl.5: **A61B  6/03**

(21) Numéro de dépôt: **88401234.5**

(22) Date de dépôt: **20.05.88**

(54) **Procédé et dispositif d'imagerie tridimensionelle à partir de mesures bidimensionnelles de l'atténuation d'un rayonnement.**

(30) Priorité: **21.05.87 FR 8707134**

(43) Date de publication de la demande:
**23.11.88 Bulletin  88/47**

(45) Mention de la délivrance du brevet:
**18.11.93 Bulletin  93/46**

(84) Etats contractants désignés:
**DE ES FR GB IT NL**

(56) Documents cités:
**FR-A- 1 071 714
FR-A- 1 195 082
FR-A- 2 394 285**

**IEEE TRANSACTIONS ON NUCLEAR SCIEN-
CE, vol. NS-26, no. 2, partie II, avril 1979,
pages 2895-2903, IEEE, New York, US; G. KO-
WALSKI: "Multislice reconstruction from
twin-cone beam scanning"**

**IEEE TRANSACTIONS ON NUCLEAR SCIEN-
CE, vol. NS-25, no. 5, octobre 1978, pages
1135-1143, IEEE, New York, US; M. SCHLIND-
WEIN: "Iterative three-dimensional reconstruction from twin-cone beam projections"**

(73) Titulaire: **COMMISSARIAT A L'ENERGIE ATO-
MIOUE
31/33, rue de la Fédération
F-75015 Paris Cédex 15(FR)**

(72) Inventeur: **Grangeat, Pierre
1, Allée Maurice Ravel
F-38130 Echirolles(FR)**

(74) Mandataire: **Mongrédien, André et al
c/o BREVATOME
25, rue de Ponthieu
F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

IEEE TRANSACTIONS ON NUCLEAR SCIEN-CE, vol. NS-26, no. 2, avril 1979, pages 2682-2684, IEEE, New York, US; G.N. MINER-BO: "Convolutional reconstruction from cone-beam projection data"

IEEE TRANSACTIONS ON BIOMEDICAL ENGI-NEERING, vol. BME-28, no. 2, février 1981, pages 79-98, IEEE, New York, US; D. NAHA-MOO et al.: "Design constraints and recons-truction algorithms for traverse-continuous-rotate CT scanners"

PHYSICS IN MEDECINE AND BIOLOGY, vol. 31, no. 3, 1986, pages 207-221, The Institute of Physics, Londres, GB; S.C. MOORE et al.: "Inversion of the 3D Radon transform for a multidetector, point-focused SPECT brain scanner"

**Description**

La présente invention concerne un procédé d'imagerie tridimensionnelle d'un objet par irradiations, ainsi qu'un dispositif étudié pour l'application de ce procédé. Une reconstruction tridimensionnelle est réalisée par le traitement d'une série de mesures bidimensionnelles de l'atténuation du rayonnement à travers l'objet, entre lesquelles on modifie l'incidence du rayonnement.

L'imagerie bidimensionnelle à partir de mesures bidimensionnelles de l'atténuation d'un rayonnement est bien connue. L'appareillage comprend une source de rayonnements appropriés, tels que les rayons X pour les examens médicaux ; l'objet à examiner est placé entre la source irradiante et un papier ou une pellicule sensible dont les points sont impressionnés en fonction de l'intensité des rayons à la sortie de l'objet ; les contrastes observés sur l'image indiquent la position des zones plus ou moins absorbantes de l'objet.

Toutefois, les informations ainsi obtenues ne sont pas suffisantes pour certaines applications, et des méthodes de reconstruction tridimensionnelle de l'objet ont été proposées.

Les méthodes de tomographie par résonance magnétique nécessitent des installations coûteuses et une très grande uniformité du champ magnétique dans lequel l'objet à examiner est placé. Par ailleurs, les temps de mesure nécessaires pour réaliser une reconstruction tridimensionnelle sont très pénalisants. Ces contraintes limitent donc leur intérêt.

On a encore proposé d'établir des reconstructions tridimensionnelles par la superposition de reconstructions bidimensionnelles ou tranches de l'objet. Une collimation d'une source de rayons X permet d'obtenir un rayonnement en éventail qui traverse une tranche de l'objet et impressionne ensuite une ligne de capteurs ; la source tourne autour de l'objet de façon à irradier la même tranche sous des angles différents ; les mesures successives sont mises en mémoire, et une calculatrice permet de déterminer la contribution locale à l'atténuation en chaque point du maillage de la tranche. La source et les capteurs sont ensuite décalés et portés sur une autre tranche autour de laquelle ils circulent selon une trajectoire parallèle à la précédente.

Le temps d'examen dépend donc du nombre de trajectoires adopté. Dans la pratique, il n'est malheureusement pas possible de se permettre un échantillonnage axial aussi serré que l'échantillonnage à l'intérieur d'une tranche ; l'objet risque également de se déplacer entre les examens de deux tranches, ce qui accroît les incertitudes de localisation.

Un autre inconvénient est lié à la collimation, qui réduit le rendement énergétique de la source et qui peut imposer des arrêts de l'appareil en cours d'examen pour permettre son refroidissement.

On a également proposé des procédés utilisant un faisceau conique qui tourne autour de l'objet et grâce auquel on réalise plusieurs irradiations qui fournissent autant d'images bidimensionnelles de l'objet. Si ces images sont suffisamment nombreuses, une calculatrice peut analyser et combiner ces images pour reconstituer une image tridimensionnelle de l'objet. Ces procédés utilisent ce qu'on appelle la transformée de Radon de l'atténuation du rayonnement en chaque point du maillage de l'objet. La transformée de Radon d'une fonction en un point est égale à l'ensemble des sommes des valeurs locales de cette fonction sur chaque plan passant par au moins un point du domaine sur lequel est délivré la fonction. On se contente évidemment en pratique d'une topologie discrète avec un nombre fini de plans pour décrire la transformée de Radon et un nombre fini de points pour décrire la fonction.

La mesure de l'atténuation du rayonnement sur une ligne de capteurs d'un détecteur plan placé derrière l'objet donne l'atténuation selon un faisceau de rayons contenus dans un plan de l'espace de Radon.

La somme de l'atténuation le long de cette ligne donne la valeur de la transformée de Radon de l'atténuation pour ce plan. L'inversion numérique de la transformée de Radon donne l'atténuation en tous points du domaine de définition de la fonction. Il faut reconnaître que l'ensemble est compliqué et que certaines des méthodes proposées conduisent à des résultats erronés ou tout du moins imprécis.

Parmi la littérature disponible, on peut citer l'article de Schlindwein "Iterative three - dimensional reconstruction from twin - cone beam projection" (IEEE Transactions on nuclear science, vol. NS-25, n°4, octobre 1978, pages 1135-1143), où les procédés utilisant la transformée de Radon sont rejetés pour leur complexité, et celui de Minerbo "Convolutional reconstruction from cone - beam projection data" (IEEE Transactions on nuclear science, vol. NS-26, n°2, avril 1979, pages 2682-2684) qui utilise un procédé mettant en oeuvre la transformée de Radon.

Comme le montre l'inventeur plus loin, l'emploi de la transformée de Radon elle-même nécessite cependant de commettre des approximations dans les calculs numériques, et d'ailleurs les articles de l'art antérieur ne fournissent pas de dispositifs concrets permettant des reconstructions de bonne qualité des images tridimensionnelles.

3

L'invention comble ces lacunes. Elle concerne d'abord des dispositifs d'imagerie tridimensionnelle comprenant tous une source unique de rayonnement conique devant l'objet et un réseau bidimensionnel de détecteur derrière l'objet, la source et le réseau étant mobiles suivant diverses incidences par rapport à l'objet. Elle concerne également un procédé mettant en oeuvre la dérivée première de la transformée de Radon de l'atténuation du rayonnement sur les points de l'objet, son calcul et son inversion.

Les échantillonnages nécessaires pour obtenir des résultats acceptables sont rappelés. Un organigramme détaillé explicitant en particulier les interpolations est proposé.

Un autre objet de l'invention est de fournir des trajectoires de la source et du dispositif de mesure autour de l'objet qui soient compatibles avec le procédé.

L'invention concerne tout d'abord un dispositif d'imagerie tridimensionnelle par irradiations d'un objet, comprenant une source de rayonnement irradiant un espace de forme conique dans lequel l'objet est placé, un détecteur comprenant un dispositif bidimensionnel mesurant l'atténuation du rayonnement ayant traversé l'objet, un mécanisme permettant d'effectuer une succession d'irradiations de l'objet sous des incidences différentes, ainsi qu'une chaîne de mesure et une calculatrice qui analysent et traitent les informations du dispositif bidimensionnel lors des irradiations pour en déduire la contribution locale en différents points d'un maillage de représentation de l'objet à l'atténuation du rayonnement, caractérisé en ce que la source est unique et en ce que la calculatrice comprend des unités programmées pour accomplir le calcul et l'inversion de la dérivée de la transformée de Radon de l'atténuation du rayonnement, la transformée de Radon d'une fonction étant définie comme l'ensemble des valeurs locales de cette fonction sur chaque plan passant par au moins un point du domaine sur lequel est définie la fonction, et la dérivée de la transformée de Radon étant définie comme la somme des taux de variation sur chacun desdits plans si on se déplace perpendiculairement audit plan dans le sens du vecteur normal défini par un système de coordonnées sphériques.

Selon un mode de réalisation possible, le mécanisme comprend un rail circulaire centré sur une origine et sur lequel la source effectue les irradiations selon une trajectoire circulaire, le détecteur se déplaçant sur cette même trajectoire et occupant des positions opposées par rapport à l'origine.

Selon un mode de réalisation plus élaboré, le mécanisme comprend deux rails circulaires parallèles, deux travées sur lesquelles coulissent respectivement la source et le détecteur, placées toutes deux avec des positions opposées par rapport à l'origine. Les travées parcourent les trajectoires circulaires quand les irradiations sont effectuées. Elles sont en outre recourbées en arc de cercle de manière à ce que la source et le détecteur restent à distance constante de l'origine.

L'invention concerne également un procédé d'imagerie tridimensionnelle d'un objet à partir de mesures bidimensionnelles de l'atténuation d'un rayonnement à travers l'objet par utilisation d'un dispositif formé d'une source de rayonnement conique comprenant un foyer et d'un détecteur bidimensionnel formé d'un réseau de capteurs, caractérisé en ce qu'on calcule en chacun des premiers points d'un maillage de représentation de l'objet l'atténuation du rayonnement par calcul de grandeurs représentatives de la dérivée de la transformée de Radon de l'atténuation du rayonnement des points d'un deuxième maillage associé à la transformée de Radon de l'objet, la transformée de Radon d'une fonction étant définie comme l'ensemble des valeurs locales de cette fonction sur chaque plan passant par au moins un point du domaine sur lequel est définie la fonction, et la dérivée de la transformée de Radon étant définie comme la somme des taux de variation sur chacun desdits plans si on se déplace perpendiculairement audit plan dans le sens du vecteur normal défini par un système de coordonnées sphériques, les grandeurs étant calculées en effectuant la sommation pour chaque deuxième point, de la variation de l'atténuation du rayonnement le long d'au moins une ligne obtenue par intersection du détecteur avec un plan passant par le foyer du rayonnement conique et à proximité du deuxième point, la droite passant par l'origine et le deuxième point étant sensiblement orthogonale au plan passant par le foyer, puis des combinaisons linéaires de ces sommations, et l'atténuation du rayonnement en chacun des premiers points étant obtenue par dérivation de ces grandeurs par rapport à la distance à l'origine, et enfin par combinaison linéaire des grandeurs dérivées, des interpolations étant effectuées par ailleurs pour passer des deuxièmes points aux premiers points.

Le procédé peut être avantageusement mis en oeuvre dans le cas où la source et le détecteur parcourent deux trajectoires à distance constante de l'origine, sensiblement en forme de sinusoïde comprenant au moins deux périodes sur un tour complet autour de l'objet et dont l'amplitude est égale ou supérieure à la distance entre l'origine et un point quelconque de l'objet, la distance entre les points des trajectoires et l'origine étant par ailleurs suffisante pour que tout plan passant par l'objet rencontre la trajectoire. Pour deux périodes, ceci est vérifié si cette distance est égale ou supérieure à cette amplitude multipliée par $\sqrt{3}$.

Le procédé de reconstruction d'images implique un mode de calcul particulier. Il consiste, pour le calcul d'un paramètre sur des premiers points d'un premier maillage tridimensionnel de points de l'objet, dont les coordonnées cartésiennes sont généralement régulièrement réparties, à tout d'abord définir des deuxièmes points et des troisièmes points qui constituent un deuxième et un troisième maillages tridimensionnels de points caractéristiques, les coordonnées sphériques des deuxièmes points étant régulièrement réparties et les deuxièmes points appartenant en particulier à des plans méridiens concourant en un axe, les troisièmes points, dont les coordonnées cylindriques sont régulièrement réparties, appartenant à la fois aux plans méridiens et à des plans parallèles contenant les premiers points et orthogonaux à l'axe ; puis à obtenir une information sur les deuxièmes points et à en calculer une information dérivée ; puis à combiner ces informations sur des groupes de deuxièmes points appartenant aux mêmes plans méridiens pour en déduire des informations intermédiaires sur les troisièmes points ; et finalement à combiner les informations intermédiaires sur des groupes de troisièmes points appartenant aux mêmes plans parallèles pour en déduire le paramètre sur les premiers points.

Les figures annexées et dont l'énumération suit permettent de mieux décrire l'invention, mais sont données à titre non limitatif :

- la figure 1 représente les principales parties d'un dispositif conforme à l'invention, ainsi que des notations utilisées pour l'explication du procédé,
- la figure 2 représente un deuxième dispositif possible selon l'invention,
- la figure 3 représente un troisième dispositif possible selon l'invention,
- la figure 4 représente une construction géométrique qui illustre une étape du procédé selon l'invention,
- la figure 5 représente une construction géométrique qui illustre une étape d'interpolation nécessaire à l'application du procédé,
- la figure 6 représente essentiellement une double trajectoire intéressante pour mener des examens à l'aide du dispositif de la figure 3,
- la figure 7 représente un schéma synoptique de l'installation qui pilote les dispositifs selon l'invention,
- les figures 8 et 9 représentent deux autres dispositifs conformes à l'invention, et
- la figure 10 représente un organigramme du procédé employé.

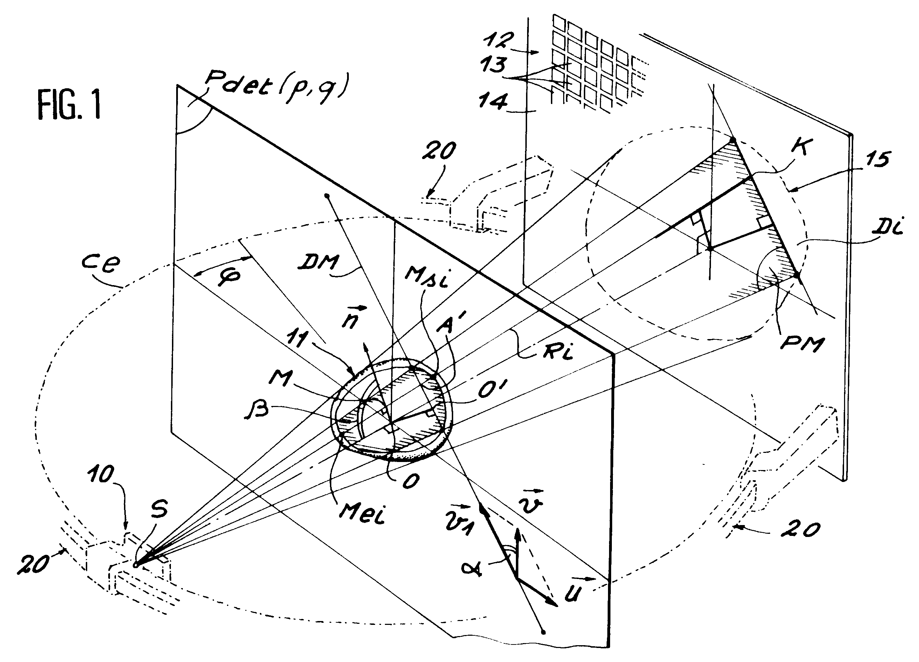

Le dispositif objet de l'invention comprend (se reporter à la figure 1) une source 10 émettrice d'un faisceau conique divergent dont le foyer est S et qui traverse avec atténuation un objet 11 à examiner et dont on souhaite reconstruire les contributions locales à l'atténuation. La nature du rayonnement peut être quelconque, par exemple des rayons X, à condition de pouvoir caractériser un composant de l'objet 11 dont on désire diagnostiquer la présence ou la concentration par une atténuation différente de celle réalisée par ses autres composants.

Un rayon individuel Ri traverse l'objet 11 entre les points d'entrée Mei et de sortie Msi ; il traverse en particulier un point intermédiaire M supposé quelconque et qui va servir de base aux raisonnements que l'on va exposer. Il atteint finalement un détecteur 12 et impressionne un capteur 13 particulier parmi ceux d'un réseau bidimensionnel sur un écran 14, situé en un point K. Il est repéré par les coordonnées (p,q) de sa trace A sur un plan de détection passant par une origine O, orthogonal à OS et référencé Pdét sur la figure 1 ; ce plan immatériel est introduit pour permettre une explication plus simple du procédé de reconstruction, sans qu'il soit nécessaire de tenir compte de l'éloignement de l'écran 14 et de sa courbure éventuelle.

Si la contribution locale au point M à l'atténuation du rayonnement est notée f(M), le capteur 13 mesure donc une intensité de rayonnement :

$$I_1 = I_0 \exp\left[-\int_{Mei}^{Msi} f(Mi)\,dMi\right],$$

l'atténuation étant supposée négligeable en dehors de l'objet 11 et $I_0$ étant l'intensité connue et fixe du rayonnement Ri qui aurait été mesurée en l'absence de l'objet 11.

$I_0$ peut aussi être fourni par un procédé de monitorage évaluant le flux direct sur des capteurs 13 du réseau bidimensionnel ou le flux localisé en dehors du champ de mesure en prise avec l'objet 11, à la sortie de la source 10.

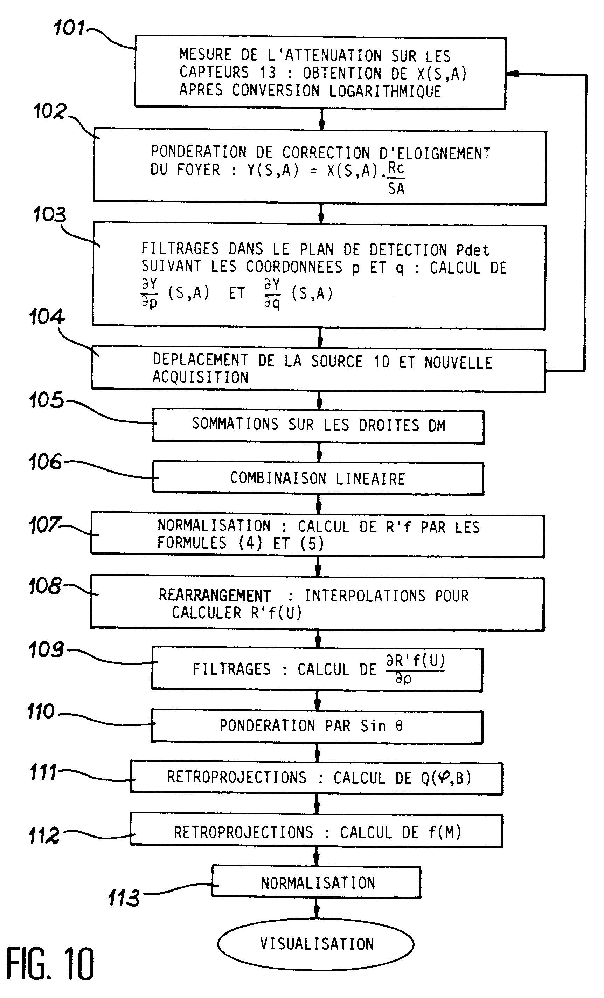

Après conversion logarithmique, on mesure donc en fait l'atténuation du rayonnement à travers l'objet le long du rayon Ri issu de S et passant par A (étape 101 de la figure 10) :

$$\int_{Mei}^{Msi} f(Mi)\,dMi = X(S,A)$$

Des coefficients d'étalonnage, calculés dans une phase initiale lors de mesures sur des objets dont l'atténuation est connue, permettent de corriger les données fournies par les capteurs.

On obtient une image bidimensionnelle 15 de l'objet 11 sur l'écran 14. La radiographie classique est formée par une telle image. Pour obtenir une reconstruction tridimensionnelle, il convient d'imprimer un mouvement tournant du foyer S de la source 10 autour de l'objet 11, par exemple sur un cercle Ce centré sur l'origine O autour de l'objet 11 de manière à l'irradier sous des incidences différentes, puis de combiner entre elles les représentations obtenues. Il est évidemment nécessaire que le détecteur 12 suive le déplacement du faisceau. Ici, on suppose qu'il se déplace également sur le cercle Ce. Il peut aussi se déplacer autrement, par exemple sur un autre cercle centré sur l'origine O mais de diamètre différent ; seul le grossissement de l'image 15 est différent. La trajectoire le long du cercle Ce peut être réalisée en accrochant la source 10 et le détecteur 12 à un rail circulaire 20, dont le diamètre peut être égal à ou différent de celui du cercle Ce en fonction du mode d'accrochage adopté.

Un problème essentiel apparaît toutefois : le volume d'information à maintenir en mémoire étant vite très important, la résolution numérique du problème de reconstruction ferait appel à des calculs matriciels compliqués. L'invention permet cependant de ne pas recourir à ce genre de procédé.

On définit tout d'abord la transformée de Radon de l'atténuation f(M) du rayonnement pour les plans passant par un point M (Rf(M)), qui définit l'ensemble des sommes de la fonction décrivant la contribution locale à l'atténuation du rayonnement en leurs points sur chacun de ces plans ; ces plans sont caractérisés chacun par un couple $(\overrightarrow{OM}.\vec{n},\vec{n})$ où n est un vecteur unitaire perpendiculaire au plan considéré et $\overrightarrow{OM}.\vec{n}$ est la mesure algébrique associée à la distance du plan à l'origine O. Pour identifier ces plans, on utilise un point caractéristique C, projection orthogonale de l'origine O sur le plan :

$$\overrightarrow{OC} = (\overrightarrow{OM}.\vec{n})\vec{n}.$$

On définit ensuite la dérivée première de la transformée de Radon de l'atténuation f(M) du rayonnement pour les plans passant par un point M (R'f(M)) qui est égale par définition, pour chaque plan $P(\overrightarrow{OM}.\vec{n},\vec{n})$, à la somme sur tous les points du plan de la dérivée de la fonction décrivant la contribution locale à l'atténuation par rapport à la direction du vecteur $\vec{n}$.

Parmi tous ces plans on distingue, et on note par abréviation PM, un plan de Radon $P(\overrightarrow{OM}.\vec{n},\vec{n})$, associé à un point caractéristique CM. $\vec{n}$ est défini dans un repère lié à l'objet 11 par sa longitude $\phi$, mesurée dans le plan du cercle Ce, et par sa colatitude $\theta$, mesurée par rapport à l'axe du cercle Ce telle que $\theta = \pi/2$ quand $\vec{n}$ appartient au plan du cercle Ce et $\theta = +0$ ou $+\pi$ quand il est perpendiculaire à ce plan.

Pour déterminer les valeurs de la transformée de Radon Rf(M) ou de sa dérivée première R'f(M), au point M, il est nécessaire de calculer en particulier la somme de la fonction décrivant la contribution locale à l'atténuation f(M) ou de sa dérivée sur tous les points du plan de Radon PM associé à ce point M. Ceci est possible si le foyer S de la source 10 appartient lui-même au plan de Radon PM, car une fraction du rayonnement ou son proche voisinage ne quitte alors pas ce plan de Radon PM ou son proche voisinage et ne peut donc pas subir d'atténuation de la part des points de l'objet 11 extérieurs au plan ou à son voisinage. Il suffit de ne considérer que cette fraction pour évaluer la somme de la fonction décrivant la contribution locale à l'atténuation ou de sa dérivée sur tous les points de l'objet 11 appartenant au plan de Radon PM (l'air ambiant n'est pratiquement pas absorbant. Si l'objet d'intérêt est contenu dans un milieu absorbant, le milieu extérieur sera considéré comme une perturbation).

Cependant, le calcul de la transformée de Radon Rf(M) ne peut être effectué ainsi que de façon approchée. L'inventeur a démontré que la dérivée première de la transformée de Radon R'f(M) est par contre calculable avec exactitude, ce qui conduit à une meilleure qualité des images tridimensionnelles.

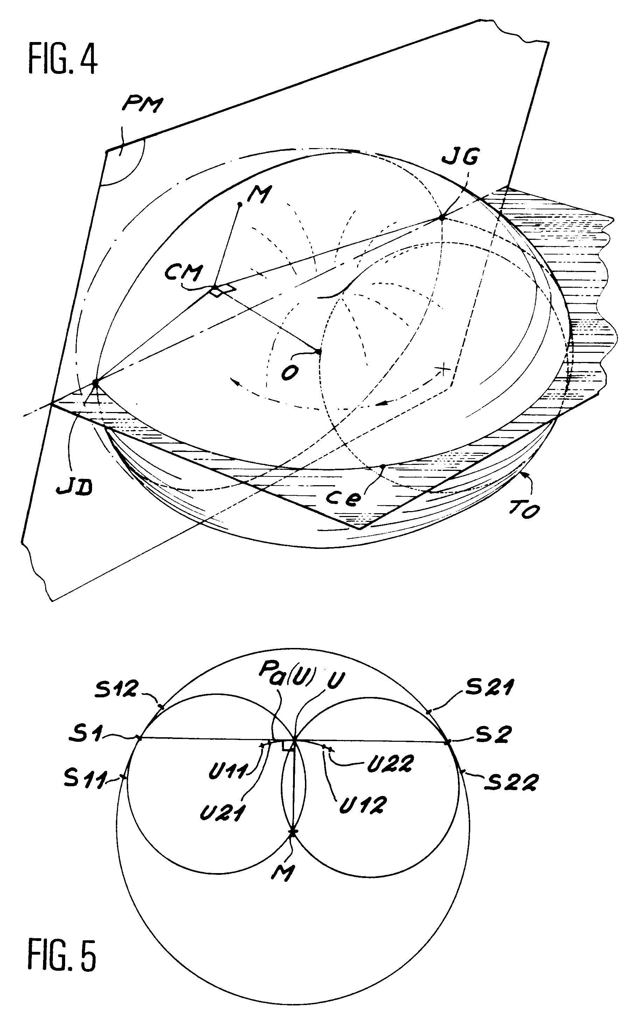

Dans le cas d'une source 10 dont le foyer S tourne autour de l'objet 11 suivant un cercle Ce, la figure 4 montre un point M dont le plan de Radon PM coupe le cercle Ce en deux points JG et JD. Le foyer S de la source 10 doit donc être placé en un de ces points pour permettre de calculer la somme de l'atténuation ou de sa dérivée sur le plan de Radon PM.

La dérivée première de la transformée de Radon R'f de l'objet lui-même est définie comme l'ensemble des sommations, sur les plans de Radon PM passant par au moins un point M de l'objet, de la dérivée, suivant le vecteur normal $\vec{n}$ de la fonction décrivant la contribution locale à l'atténuation. Le plan de Radon PM est commun à tous les points de l'objet 11 qu'il contient et en particulier au point caractéristique CM. On associe ici à ce point CM la valeur de la transformée de Radon Rf(M) ou de sa dérivée R'f(M) sur le plan PM.

On appelle volume caractéristique de l'objet l'ensemble des points caractéristiques CM associés à tous les plans de Radon PM passant par au moins un point M de l'objet. La reconstruction de l'image tridimensionnelle peut être effectuée quand on dispose de l'atténuation sur l'ensemble de ces plans.

Cependant, les mesures ne permettent d'accéder à une valeur de R'f que pour les plans PM rencontrant la trajectoire parcourue par le foyer S. On appelle volume caractéristique des mesures l'ensemble des points caractéristiques CM associés aux plans de Radon passant par au moins un point de la trajectoire. Le volume caractéristique de l'objet doit autant que possible être inclus dans le volume caractéristique des mesures.

Dans le cas d'un objet sphérique centré sur l'origine 0 et de rayon Rob, et de la trajectoire circulaire Ce, le volume caractéristique de l'objet est la même sphère centrée sur O et de rayon Rob. Le volume caractéristique des mesures est un tore To, représenté sur la figure 4, obtenu par rotation d'un cercle contenu dans un plan passant par le foyer S et l'axe du cercle Ce, tangentant l'axe au niveau de l'origine O et de diamètre SO.

On s'aperçoit ainsi que le volume caractéristique des mesures ne permet pas de couvrir tout le volume caractéristique de l'objet : il reste une zone d'ombre caractéristique des plans qui rencontrent l'objet mais pas le cercle Ce. Le calcul de R'f sur cette zone ne pourra se faire que par interpolation.

Quelle que soit la position de l'objet, il existera toujours une zone d'ombre liée aux plans qui passent par l'objet mais ne rencontrent pas le cercle Ce. Pour combler cette zone d'ombre, il faut renoncer à la trajectoire circulaire simple et choisir une trajectoire telle que tout plan passant par au moins un point de l'objet rencontre la trajectoire.

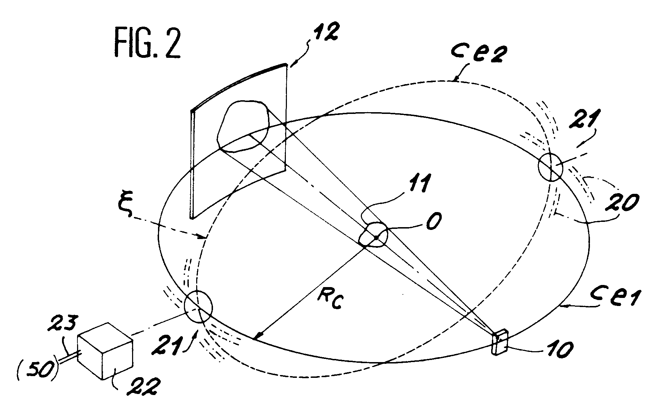

Cette condition peut être concrètement remplie, si la trajectoire circulaire Ce est produite par le déplacement concomitant de la source 10 et du détecteur 12 le long d'un rail circulaire 20 et si on associe (figure 2) audit rail 20 un mécanisme de pivotement 21 d'un angle $\xi$ suivant un axe passant par l'origine O, lui permettant ainsi de prendre deux positions matérialisées par les cercles Ce1 et Ce2. Un blocage peut être prévu, par tout moyen approprié, pour ces deux positions. On peut facilement se rendre compte que, plus l'angle $\xi$ est proche de $\pi/2$, plus le rayon maximal Rob de la sphère objet centrée sur O, dont le volume caractéristique ne possède pas de zone d'ombre, augmente. Pour $\xi$ valant $\pi/2$, c'est-à-dire pour deux trajectoires perpendiculaires, la valeur maximum pour Rob est $RC/\sqrt{2}$ si Rc désigne le rayon des cercles Ce1 ou Ce2.

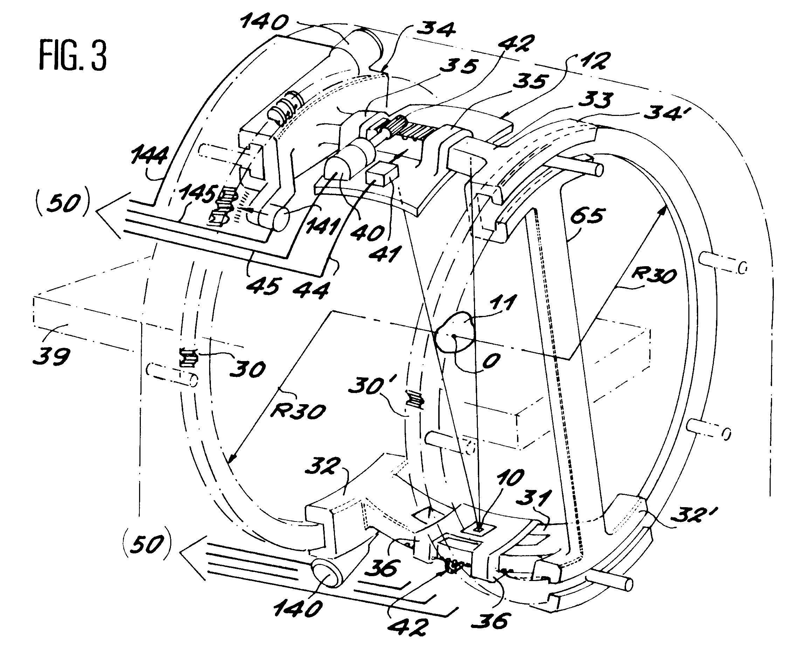

On représente figure 3 une autre possibilité de réaliser une telle trajectoire : le dispositif comprend ici deux rails parallèles circulaires 30 et 30′ de même rayon $R_{30}$ parcourus chacun par deux supports diamétralement opposés 32 et 34, et 32′ et 34′ respectivement. Les supports 32 et 32′ constituent les extrémités d'une travée 31 le long de laquelle la source 10 coulisse par des poignées 36 ; de même, les supports 34 et 34′ constituent les extrémités d'une travée 33 le long de laquelle le détecteur 12 coulisse par des poignées 35. Les travées 31 et 33 sont en arcs de cercles centrés sur l'origine O.

On peut donc combiner deux rotations pour la source 10 et le détecteur 12 pour les déplacer sur des trajectoires appartenant à deux sphères concentriques. Nous les supposons par la suite identiques.

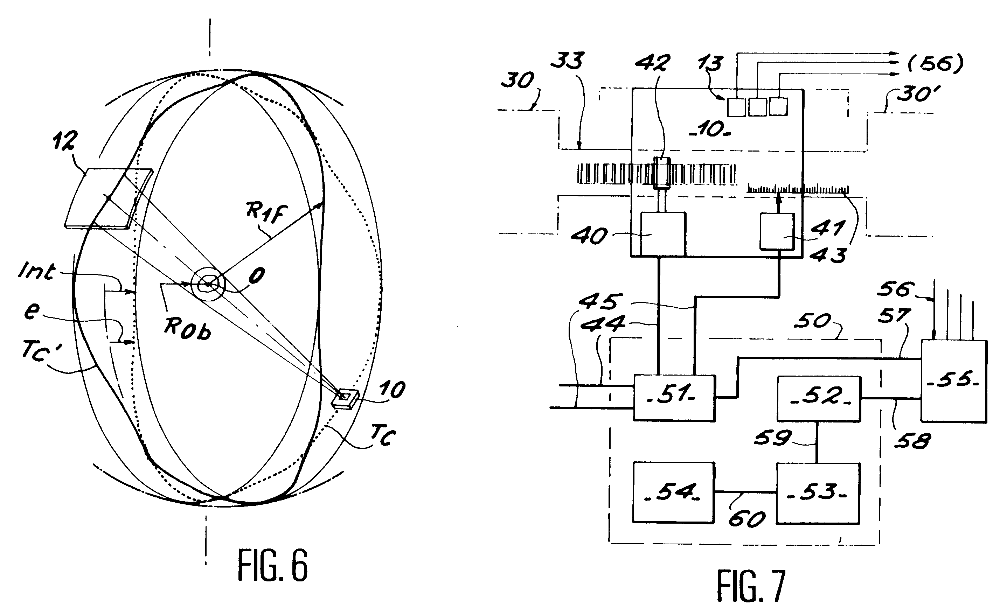

Un exemple possible est celui où le foyer S de la source 10 parcourt une trajectoire Tc représentée figure 6 et d'équation $e = Int.\cos 2\psi$, où $\psi$ désigne un angle de rotation le long des rails 30 et 30′, e un débattement parallèlement à l'axe des rails 30 et 30′, avec $e = o$ quand le foyer S appartient au plan passant par l'origine O et parallèle aux rails 30 et 30′, et Int une amplitude. On peut alors démontrer que la transformée de Radon Rf(M) ou sa dérivée R'f(M) peuvent être obtenues pour tous les points CM caractéristiques de l'objet 11 si $R1f \geqq Int. \sqrt{3}$ et $Int \geqq Rob$, où R1f est le rayon de la sphère support de la trajectoire Tc et Rob est le rayon de la plus petite sphère centrée sur l'origine O et contenant tout l'objet 11.

Au cours du déplacement de la source 10, le point d'attache du détecteur 12 se déplace en concomitance sur les points d'une trajectoire Tc′ symétrique par rapport à l'origine O.

L'avantage du dispositif de la figure 3 par rapport à celui de la figure 2 est de permettre d'effectuer en une seule rotation continue les mesures alors que la double trajectoire circulaire impose une double rotation et un temps d'arrêt pour le pivotement et par là-même, pour une vitesse de précession donnée, un temps d'examen au moins deux fois plus grand. Par ailleurs, la double trajectoire circulaire introduit une redondance importante dans les mesures puisqu'il existe une forte proportion de plans qui rencontrent à la fois les deux trajectoires. Une alternative au dispositif de la figure 3 consiste à munir les points 21 de la figure 2 d'un moteur 22 commandé par une calculatrice 50 (figure 7) par l'intermédiaire d'une ligne 23 de façon à faire varier l'angle ξ quand le foyer S se déplace sur le cercle Ce. Une trajectoire quelconque peut être obtenue dans l'espace tridimensionnel. Vu les masses respectives des sources de rayonnement X et des détecteurs bidimensionnels tels que les amplificateurs de brillance, le dispositif de la figure 3 semble préférable.

On a également représenté figure 3 le dispositif qui commande le déplacement du détecteur 12 sur la travée 33. Il comprend un moteur électrique 40 relié à une calculatrice 50 (figure 7) par une ligne 44 et dont l'arbre est terminé par un pignon 42 qui s'engrène dans une face en crémaillère de la travée 33 ; comme les mouvements demandent une grande précision, une autre face de la travée 33 comporte des graduations 43 qu'un capteur optique 41 repère ; il envoie alors un signal à la calculatrice 50 par une ligne 45, et l'arrêt du moteur 40 est commandé par la ligne 44. Un moteur et un capteur optique identiques commandent également le déplacement de la source 10 sur la travée 31 ; il existe d'autre part des dispositifs analogues à moteur électrique 140 et capteur optique 141, reliés respectivement à la calculatrice 50 par des lignes 144 et 145, pour déplacer les supports 32, 32′ et 34, 34′ le long des rails 30, 30′ qui comportent donc également une crémaillère et des graduations.

Les dispositifs des figures 1 et 2 peuvent tout aussi bien être pilotés par ces dispositifs quoiqu'ils n'aient pas alors été représentés.

Les déplacements de la source et de l'écran peuvent, d'une façon générale, être indépendants et synchronisés ; ils peuvent aussi être obtenus à l'aide d'un seul moteur et d'une liaison mécanique telle que réalisée par une barre ou, plus généralement, une structure mécanique rigide.

D'autres dispositifs sont en effet possibles pour étudier l'objet 11.

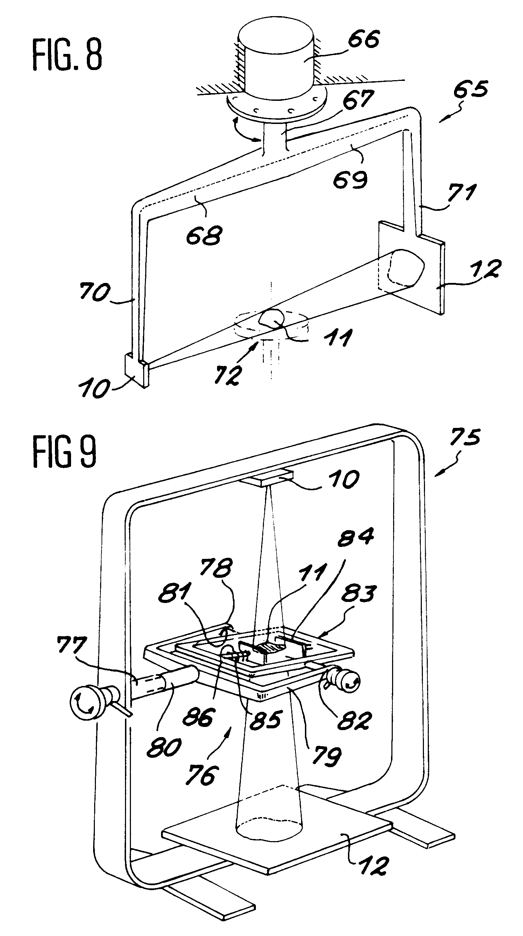

Tout d'abord, comme le représente la figure 8, on peut envisager un dispositif impliquant un déplacement conjoint de la source 10 et du détecteur 12, reliés par une structure mécanique rigide 65 tournant sous l'action d'un moteur 66 commandé par la calculatrice 50, autour de l'objet 11. Cette structure comprend un montant vertical 67 qui est également l'arbre de sortie du moteur 66 et d'où partent deux bras radiaux 68 et 69 opposés. Le premier bras 68 se termine par une première perche 70 à laquelle la source 10 est suspendue, le deuxième bras 69 par une deuxième perche 71 à laquelle le détecteur 12 est suspendu. Dans cette réalisation comme dans les précédentes, l'objet 11 est posé sur un support transparent au rayonnement représenté ici par 72.

Comme le montre la figure 9, on peut encore appliquer l'invention à un dispositif dans lequel la source 10 et le détecteur 12 sont fixes et accrochés à une structure immobile 75 et, sur cette figure, représentés respectivement en haut et en bas de l'objet 11.

L'objet 11 est mû en rotation à l'aide d'une fourche 76 dont le manche 80 tourne dans un palier 77 ménagé dans la structure 75, autour d'un axe sensiblement perpendiculaire au rayonnement émis par la source 10. Deux paliers, référencés respectivement 81 et 82, sont ménagés dans les deux branches 78 et 79 de la fourche 76 et reçoivent deux pivots situés de part et d'autre d'un cadre plan 83, qui tourne donc entre les deux branches 78 et 79 autour d'un axe perpendiculaire à l'axe de rotation de la fourche 76.

L'objet 11 est fixé rigidement, par compression par exemple, entre deux plaques de compression 84 à une extrémité de tiges télescopiques 85 dont l'autre extrémité est solidaire du cadre 83 ; les tiges télescopiques 85 ont une directrice commune et se déploient l'une vers l'autre, poussées par des ressorts 86 autour ou dans les tiges et comprimés entre le cadre 83 et la plaque de compression 84 respective au cadre 83. On peut donc le soumettre à une rotation quelconque et accomplir les mêmes examens qu'avec les autres dispositifs décrits jusqu'ici. Les plaques de compression et éventuellement les tiges sont supposées transparentes au rayonnement de la source 10.

Les mouvements de rotation de la fourche 76 et du cadre 83 sont commandés par la calculatrice 50 à l'aide de moteurs non représentés.

Ce dispositif n'est pas adapté à l'examen d'un être humain, mais présente de l'intérêt pour de petits objets en contrôle non destructif. Il faut toutefois limiter les rotations de manière à éviter au rayonnement de traverser le cadre 83 et les branches 78 et 79, ou bien prévoir également ces pièces en matériau transparent.

Pour décrire le procédé de reconstruction de l'image à l'aide de la dérivée R'f de la transformée de Radon, on est amené à définir de nouvelles notations (figure 1). On apppelle plan de détection Pdét associé à la position du foyer S de la source 10, le plan perpendiculaire à l'axe OS et passant par l'origine 0. Ce plan est muni d'un repère cartésien $(\vec{u},\vec{v})$ tel que le repère $(\vec{u},\vec{v},$

$$\frac{\overrightarrow{OS}}{||\overrightarrow{OS}||})$$

soit direct. Le vecteur $\vec{u}$ est choisi parallèle au plan du cercle Ce. Ce plan de détection Pdét sert à définir les coordonnées p et q des capteurs 13 sur l'écran de détection 14. On appelle droite de sommation D-$(\overrightarrow{OM}.\vec{n},\vec{n})$ ou DM l'intersection du plan de Radon $P(\overrightarrow{OM}.\vec{n},\vec{n})$ ou PM associé au point M avec le plan de détection, et O' le point de DM projection orthogonale du point O sur la droite DM. La droite DM est orientée par un vecteur unitaire $\vec{v}_1$ tel que :

$$(\vec{n},\ \vec{v}_1,\ \frac{\overrightarrow{O'S}}{||\overrightarrow{O'S}||})$$

forme un repère direct. On appelle $\alpha$ l'angle entre les vecteurs $\vec{v}$ et $\vec{v}_1$.

Il faut aussi définir les deux fonctions d'atténuation pondérée :

$$Y(S,A) = X(S,A) \cdot \frac{Rc}{SA}$$

$$Z(S,A) = X(S,A) \cdot \left(\frac{Rc}{SA}\right)^3$$

où Rc est le rayon de la trajectoire sur laquelle se déplace le foyer S de la source 10, c'est-à-dire la distance OS. Ces fonctions sont indépendantes du plan de Radon considéré.

On leur associe pour chaque droite DM les fonctions SY(S,M) et SZ(S,M) représentant respectivement l'intégrale des fontions Y et Z sur la droite de sommation.

On démontre, ce qui est le point de départ des calculs numériques, que :

$$(1) \qquad \frac{||\overrightarrow{OS}||}{||\overrightarrow{OS}\wedge\vec{n}||} \cdot SZ(S,M) \approx Rf(M)$$

$$(2) \qquad \frac{1}{||\overrightarrow{OS}||} \cdot \frac{\partial SY(S,M)}{\partial\beta} = -R'f(M)$$

ou $\beta$ représente l'angle entre les vecteurs $\vec{n}$ et $\overrightarrow{OS}$.

La première formule (1) est une relation approchée liant les mesures à la transformée de Radon Rf. La deuxième (2) est une relation exacte liant les mesures à la dérivée première de la transformée de Radon R'f ; elle permet d'obtenir des résultats plus précis et reste vérifiée quelle que soit la distance entre la source 10 et l'objet 11. Elle permet donc de s'en rapprocher au maximum, alors que l'approximation de la formule (1) est d'autant meilleure que la source 10 est éloignée de l'objet 11, ce qui impose un dispositif plus encombrant.

La formule (2) est mathématiquement équivalente, si on pose :

$$(3) \qquad Y'(S,A) = \cos\alpha\ \frac{\partial Y}{\partial p}(S,A) + \sin\alpha\ \frac{\partial Y}{\partial q}(S,A),$$

aux deux formules :

$$(4) \quad R'f(M) = \frac{||\vec{OS}||^2}{||\vec{OS} \wedge \vec{n}||^2} \cdot \frac{1}{|\sin \alpha|} \cdot \int_{p=-\infty}^{+\infty} Y'(S,A(p))dp$$

$$(5) \quad R'f(M) = \frac{||\vec{OS}||^2}{||\vec{OS} \wedge \vec{n}||^2} \cdot \frac{1}{|\cos \alpha|} \cdot \int_{q=-\infty}^{+\infty} Y'(S,A(q))dq$$

où A(p), respectivement A(q), représente le point A de la droite de sommation DM d'abscisse p, respectivement d'ordonnée q.

On choisira de préférence la formule (5) pour $\alpha$ proche de O modulo $\pi$, et la formule (4) pour $\alpha$ proche de $\pi/2$ modulo $\pi$.

Comme le montre l'organigramme de la figure 10, on commence donc par calculer, pour une position donnée de la source 10, les quantités X(S,A) et Y(S,A) de tout point A du plan de détection Pdét (étapes 101 et 102). Les dérivées de Y(S,A) par rapport aux coordonnées p et q du plan de détection Pdét sont accomplies par des opérations de filtrage (étape 103) en utilisant la convolution de la fonction Y(S,A) par deux filtres de différentiation. La source 10 est ensuite déplacée jusqu'à sa position suivante (étape 104) et le cycle recommence jusqu'à ce que toutes les acquisitions aient été effectuées.

Les étapes suivantes consistent à effectuer des calculs pour des points caractéristiques U' du volume caractéristique de l'objet. Ces calculs font intervenir le plan de Radon PM qui leur est associé et la droite de Radon DM intersection du plan de Radon PM avec le plan de détection Pdét. On commence par calculer (étape 105) :

- dans le cas de la formule (4) :

$$\int_{p=-\infty}^{p \; +\infty} \frac{\partial Y}{\partial p}(S,A) \, dp \quad \text{et} \quad \int_{p=-\infty}^{+\infty} \frac{\partial Y}{\partial q}(S,A) \, dp, \quad \text{et}$$

- dans le cas de la formule (5) :

$$\int_{q=-\infty}^{+\infty} \frac{\partial Y}{\partial p}(S,A) \, dq \quad \text{et} \quad \int_{q=-\infty}^{+\infty} \frac{\partial Y}{\partial q}(S,A) \, dq,$$

par sommation sur les droites de Radon DM, puis une combinaison linéaire (étape 106) permet d'obtenir :

- dans le cas de la formule (4) :

$$\cos\alpha \int_{p=-\infty}^{+\infty} \frac{\partial Y}{\partial p}(S,A)\,dp + \sin\alpha \int_{p=-\infty}^{+\infty} \frac{\partial Y}{\partial q}(S,A)\,dp, \text{ et}$$

- dans le cas de la formule (5) :

$$\cos\alpha \int_{q=-\infty}^{+\infty} \frac{\partial Y}{\partial p}(S,A)\,dq + \sin\alpha \int_{q=-\infty}^{+\infty} \frac{\partial Y}{\partial q}(S,A)\,dq, \text{ et}$$

après quoi ces quantités sont normalisées (étape 107) en les multipliant par :

$$\frac{||\overrightarrow{OS}||^2}{||\overrightarrow{OS}\wedge\overrightarrow{n}||^2},$$

puis par 1/|sin α| ou 1/|cosα| respectivement. Le résultat est égal à R'f(U).

Si le système de coordonnées (p,q) n'est pas adapté à l'échantillonnage fixé par la répartition des capteurs sur le détecteur 12, on peut éventuellement transposer ces formules dans le système de coordonnées approprié. On peut aussi à l'inverse recalculer par interpolation les atténuations dans le système de coordonnées (p,q), effectuant ainsi ce qu'on peut appeler un redressement ou un rééchantillonnage des mesures.

Dans le cas où un plan de Radon rencontre la trajectoire en plusieurs points, par exemple deux (JG et JD) dans le cas de la figure 4, on a intérêt à effectuer une moyenne sur toutes ou au moins sur une partie des valeurs de R'f associées à chacun de ces points afin de réduire les erreurs statistiques liées au bruit sur les mesures. De façon générale, on choisit cependant une moyenne sur deux points privilégiés.

Dans le cas où un plan de Radon associé à un point caractéristique CM ne rencontre la trajectoire en aucun point, ce qui arrive si le volume caractéristique des mesures laisse une zone d'ombre dans le volume caractéristique de l'objet, il est souhaitable de lui attribuer quand même une valeur R'f par une procédure d'interpolation. Pour cela on peut par exemple mettre en place une interpolation d'ordre zéro : on associe au point caractéristique CM hors du volume caractéristique des mesures la surface correspondant à l'intersection de la sphère centrée sur O et passant en CM avec le volume caractéristique des mesures. On choisit alors sur cette surface un point C'M (non représenté) à distance minimale de CM. Par définition du volume caractéristique des mesures, le plan P'M (non représenté) admettant comme point caractéristique C'M va rencontrer la trajectoire en un ou plusieurs points, la plupart du temps en lui restant tangent. La méthode précédente permet de définir une valeur R'f associée à C'M. L'interpolation d'ordre zéro consiste à attribuer cette même valeur au point CM.

On arrive donc à attribuer une valeur R'f à tous les points du volume caractéristique de l'objet. Comme il a déjà été dit, il est bien entendu préférable d'éviter l'interpolation sur la zone d'ombre et donc d'utiliser un dispositif tel que celui représenté sur la figure 3. Cependant, pour plus de simplicité mécanique du dispositif, on peut être amené à se contenter de la simple trajectoire circulaire Ce et tolérer l'interpolation pour fixer les valeurs sur la zone d'ombre associée.

Il reste à déduire f(M) de la connaissance de la dérivée première R'f de la transformée de Radon sur le volume caractéristique objet. Cette opération d'inversion est simple et peut être mise en oeuvre par des calculs performants.

Pour un vecteur unitaire $\vec{n}$ donné, on note $\rho$ la mesure algébrique (rayon) des points U′ sur l'axe d'origine O et de vecteur directeur $\vec{n}$.

La formule théorique d'inversion de la dérivée première de la transformée de Radon R′f s'écrit :

$$(6) \qquad f(M) = -\frac{1}{4\pi^2} \int_{\varphi=0}^{\pi} \int_{\theta=0}^{\pi} \frac{\partial R'f}{\partial \rho}(U') \sin\theta\, d\theta\, d\varphi$$

On rappelle que $\phi$ et $\theta$ désignent la longitude et la colatitude du point considéré (ici U′), et $\rho$ est défini par $\vec{OU'} = \rho\vec{n}$.

Il est toutefois nécessaire de se pencher sur les problèmes de discrétisation, qui n'ont pas été évoqués jusqu'ici.

Il est évident en particulier que la source 10 ne peut effectuer des expositions que sous un nombre fini d'incidences déterminées, que l'écran de détection 14 effectue les mesures à l'aide d'un réseau de capteurs 13 repéré sur le plan de détection en nombre fini également, et que l'objet 11 doit être discrétisé ou maillé lui aussi.

Des problèmes d'interpolation se posent inévitablement. Les solutions préconisées, et qui appartiennent également à l'invention, sont décrites ci-dessous. On examine simultanément les conditions auxquelles les discrétisations doivent répondre pour permettre d'obtenir des reconstructions correctes.

L'objet 11, supposé entièrement inclus dans une sphère centrée sur l'origine O et de rayon Rob, peut être décrit à l'aide des points M qui résultent d'une discrétisation sur un maillage de représentation ou de visualisation parallélépipédique régulier dont les coordonnées vérifient :

x(i) = [(2i-1-Nx)/Nx].Rob où 1≦i≦Nx,
y(j) = [(2j-1-Ny)/Ny].Rob où 1≦j≦Ny,
z(k) = [(2k-1-Nz)/Nz].Rob où 1≦k≦Nz.

Si $\gamma$ est la fréquence de coupure recherchée pour le système d'imagerie 3D, on prend de préférence Nx, Ny et Nz supérieurs ou égaux à 4.$\gamma$.Rob.

L'écran de détection 14 est également avantageusement gradué en coordonnées cartésiennes, que l'on a d'ailleurs déjà introduites. Les emplacements des capteurs 13 définis par leurs coordonnées sur le plan de détection Pdét vérifient :

p(a) = [(2a-1-Np)/(Np-2)].Rob où 1≦a≦Np,
q(b) = [(2b-1-Nq)/(Nq-2)].Rob où 1≦b≦Nq.

Np et Nq sont égaux ou supérieurs à 4.$\gamma$.Rob.

Les points U de l'échantillonnage de la dérivée première de la transformée de Radon sont définis à l'aide d'un maillage sphérique centré sur l'origine O ; leurs coordonnées sphériques vérifient :

rayon : $\rho$(n) = [(2n-1-Nn)/(Nn-2)].Rrad où 1≦n≦Nn,
colatitude : $\theta$(1) = [(21-1)/2Nl].$\pi$/2 où 1≦1≦Nl,
longitude : $\phi$(m) = [(m-1)/Nm].2$\pi$ où 1≦m≦Nm.

où Rrad est le rayon de la sphère centrée sur O englobant le volume caractéristique objet. Pour une sphère objet de rayon Rob, Rrad = Rob.

On prend de préférence pour Nn un nombre pair pour ne pas incorporer l'origine O dans le maillage, car elle caractérise une infinité de plans. D'autre part on choisit :

Nm = 2.Nn et Nl = Nn/2 pour 2$\pi$.$\gamma$.Rob≦NM≦4$\pi$.$\gamma$.Rob.

EP 0 292 402 B1

Ceci permet de réduire les artéfacts liés à l'inversion de la dérivée première de la transformée de Radon à un niveau acceptable.

Les positions de la source 10 à partir desquelles on réalise les mesures d'atténuation sont les intersections des plans méridiens orthogonaux aux plans méridiens de longitude $\phi(m)$ avec la trajectoire Ce ou Tc.

Le problème concret consiste donc à calculer, à partir des Nm positions que peut prendre la source 10, la dérivée première de la transformée de Radon R'f(U) pour tout le réseau des points U, et ensuite les contributions locales à l'atténuation f(M) pour tout le réseau des points M. On y parvient à l'aide d'une succession d'étapes d'interpolation. Un procédé possible est exposé dans la suite du texte.

Pour les points U faisant partie du volume caractéristique de mesure, la figure 5 montre que la dérivée de la transformée de Radon R'f(U) de tout point U peut être obtenue pour au moins deux emplacements généralement différents S1 et S2 du foyer S, pour lesquels les sphères dont les diamètres sont limités par l'origine O et, respectivement, par les emplacements $S_1$ et $S_2$, se rencontrent au point U. Dans la pratique les emplacements S1 et S2 ne correspondent cependant pas à des positions de mesure du foyer S mais sont situés chacun entre deux de ces positions, S11 et S12, et S21 et S22 respectivement. Pour les points U extérieurs au volume caractéristique de mesure, on repère les positions de source $S_1$ et $S_2$ à partir du point du volume caractéristique de mesure servant à définir l'interpolation d'ordre zéro présentée précédemment.

On calcule en fait la dérivée première de la transformée de Radon pour les points U11, U12, U21 et U22 faisant partie de l'ensemble des points U' et étant les points d'intersection les plus proches de U des sphères de diamètres respectifs OS11, OS12, OS21 et OS22 avec le cercle correspondant au parallèle du point U (ensemble des points de même rayon $\rho$ (n) et de même colatitude $\theta(l)$). Si cette intersection est vide, on prend les points des cercles d'intersection entre ces sphères et la sphère centrée sur O et passant par U les plus proches du cercle correspondant au parallèle du point U. Leurs distances du point U sont respectivement d11, d12, d21 et d22.

On calcule la transformée de Radon du point U ou sa dérivée par la formule d'interpolation (7) :

$$R'f(U) = \frac{1}{2}\left(\frac{d12R'f(U11)+d11R'f(U12)}{d11+d12} + \frac{d22R'f(U21)+d21R'f(U22)}{d21+d22}\right) \text{ et}$$

Cette opération correspond à l'étape 108 de l'organigramme.

Il est évident que la calculatrice qui gère le processus connaît à l'avance la position des points S11, S12, S21, S22, U21, U12, U21, U22 pour tout point U de l'échantillonnage, ainsi que les distances d11, d12, d21 et d22 qui sont stockées en mémoire ; la position des droites de sommation associées aux points U11, U12, U21 et U22 est également connue à l'avance et les valeurs de l'atténuation X(S,A) le long de ses points sont obtenues par interpolation des valeurs mesurées sur les capteurs 13 traversés par cette droite. Pour calculer la somme sur les points considérés d'une droite de sommation quelconque, la calculatrice 50 possède donc des coefficients pondérateurs associés à chaque capteur 13. Avec l'échantillonnage proposé, les points U sont au nombre de NlxNmxNn = $Nn^3$ et il y a 2.Nn positions de la source 10 ; pour chacune de ces positions on calcule la transformée de Radon ou sa dérivée pour $Nn^2$ points U, c'est-à-dire qu'on effectue des sommations le long de $Nn^2$ droites de sommation DM. Puis on calcule une moyenne sur les valeurs regroupées deux par deux.

Pour exécuter le calcul numérique de la formule (6), la séparation des intégrales est possible et avantageuse.

Pour décrire l'exécution de ce calcul, on introduit la notion de plan de projections réarrangées, passant par l'origine O et dont les points sont à longitude $\phi$ constante. Il s'agit donc de plans méridiens.

A tout point B de ce plan et à tout vecteur $\vec{n}$ de longitude $\phi$ on associe le point CB, projection orthogonale du point B sur l'axe passant par l'origine O et de vecteur directeur $\vec{n}$. Quand B décrit le plan de projections réarrangées, le point CB décrit un plan de points caractéristiques correspondant au plan méridien associé à la longitude $\phi$. Géométriquement ces deux plans sont confondus.

On peut remarquer que si B est la projection orthogonale du point M sur le plan de projections réarrangées, le point CB est identique au point caractéristique CM associé au plan PM défini par M et $\vec{n}$.

On peut donc commencer par calculer sur chaque plan de projection réarrangées, à partir des valeurs R'f sur le plan méridien associé, la quantité :

13

$$(8) \quad Q(\varphi,B) = - \frac{1}{4\pi^2} \int_{\theta=0}^{\pi} \frac{\partial R'f}{\partial \rho}(CB) \sin\theta d\theta$$

et ce pour tous les points B qui sont projections orthogonales d'au moins un point M de l'objet.

Puis, dans un deuxième temps, on calcule la contribution locale à l'atténuation pour chaque point M de l'objet :

$$(9) \quad f(M) = \int_{\varphi=0}^{\pi} Q(\varphi,B)d\varphi$$

Cette méthode permet d'obtenir une reconstitution rigoureuse de l'objet 11 pour peu que le volume caractéristique de l'objet soit entièrement inclus dans le volume caractéristique de mesures.

Par ailleurs, l'utilisation de la formule exacte permet de rapprocher la source 10 de l'objet 11. On peut ainsi réduire l'encombrement du dispositif et augmenter le facteur de grossissement (distance foyer S - détecteur 12/distance foyer S - objet 11) et ainsi améliorer la résolution spatiale du dispositif. De plus, cela contribue à une meilleure utilisation du rayonnement dans la mesure où cela élargit l'angle solide d'irradiation de l'objet. Comme les sources de rayonnement sont limitées dans leur débit de photons par unité d'angle, cela permet, pour un nombre de photons total devant passer par l'objet pendant la durée des mesures, de réduire le temps d'examen et donc d'accroître la résolution temporelle. Cela permet aussi en conservant le même temps d'examen, d'améliorer la précision statistique sur l'objet reconstruit.

Le schéma de reconstruction peut être généralisé à une large classe de trajectoires en autorisant par exemple que l'éloignement du foyer de la source 10 ou du détecteur 12 soit différent, voire même variable. Dans les formules précédentes, les coefficients de pondération, liés au grossissement dépendront alors des mesures.

Il faut remarquer que la source 10 et l'objet 11 peuvent également être rapprochés si on choisit une trajectoire selon la figure 6 et conforme à la formule :

$$e = Int(\cos.n\psi)$$

et ce d'autant plus que n est élevé.

L'inversion de la dérivée de la transformée de Radon, définie par les formules vues plus haut, s'effectue concrètement à l'aide des points B d'un maillage dit de réarrangement tracé sur les plans de projections réarrangées associées aux méridiens des points U, par l'échantillonnage :

$r(c) = [(2c-1-Nn)/(Nn-2)].Rrad$ où $1{\leq}c{\leq}Nn$,
$z(d) = [(2d-1-Nz)/Nz].Rob$ où $1{\leq}d{\leq}Nz$,
$\phi(m) = [(m-1)/Nm].2\pi$ où $1{\leq}m{\leq}Nm$,

où $r(c)$ désigne la coordonnée d'un point B d'un plan méridien suivant l'axe parallèle au plan du cercle Ce, et $z(d)$ la coordonnée d'un point de réarrangement B suivant l'axe de rotation du cercle Ce : sur chaque plan méridien, les points B sont régulièrement répartis en un réseau rectangulaire.

Le calcul de la quantité $Q(\phi,B)$ telle qu'elle a été définie plus haut se limite donc en fait au calcul des quantités $Q(\phi,B)$ pour les points du maillage de réarrangement après qu'une interpolation a permis d'obtenir les dérivées $\partial R'f(CB)/\partial\rho$ à partir des dérivées $\partial R'f(U)/\partial\rho$, pour chaque direction du méridien (étape 111).

Le calcul des dérivées $\partial R'f/\partial\rho$ aura avantageusement été effectué par des techniques de traitement numérique, code par exemple la convolution par les filtres associés. Les données auront aussi été pondérées au préalable par le facteur $\sin\theta$ (étapes 109 et 110 de filtrages et pondération).

Suivant des techniques connues, les opérations appelées de filtrage, liées au calcul de $\partial R'f/\partial\rho$, et de rétroprojection liées à la sommation sur la colatitude $\theta$ peuvent être remplacées par une opération de rétroprojection suivie d'une opération de filtrage sans sortir du cadre de la présente invention. L'opération de filtrage pourra aussi éventuellement intervenir sur l'objet 11 après que la sommation sur les angles $\theta$ et $\phi$ a été effectuée.

Les dernières interpolations à réaliser s'effectuent à l'intérieur de chaque plan de l'objet perpendiculaire aux plans méridiens : on a vu que le calcul de la contribution locale à l'atténuation f(M) du point M fait intervenir les projections orthogonales du point M sur chaque plan de projection réarrangées pris en compte. Ces projections tombent entre les points B dont il faut donc interpoler les quantités Q($\phi$,B) avant de les sommer, réalisant ainsi des opérations de rétroprojection (étape 112).

Pour ces dernières opérations, le système connaît également à l'avance les positions des points des différents maillages et possède, sous forme de tables ou de matrices, les coefficients à prendre en compte. Le travail de programmation préalable est donc important et il doit être répété si on veut disposer de plusieurs échantillonnages différents, mais les calculs à effectuer au cours d'un examen restent raisonnables et comprennent essentiellement des combinaisons linéaires et des filtrages.

Une calculatrice 50 gère le processus de reconstruction (figure 7). Elle se compose d'une unité de synchronisation 51, d'une unité de mémoire 52, d'une unité de calcul 53 et d'unités périphériques 54. Dans un premier temps, l'unité de synchronisation 51 assure l'acquisition des mesures : en fonction des indications des capteurs optiques 41 ou 141, elle met en action les moteurs électriques 40 ou 140 et les arrête quand la source 10 et le détecteur 12 sont à une position prédéterminée de mesure ; elle indique alors, par l'intermédiaire d'une ligne 57, le début et la fin des acquisitions à une chaîne de mesures 55 ordinaire reliée par une ligne 56 à chacun des capteurs 13 dont les informations traitées sont fournies par une ligne 58 à l'unité de mémoire 52.

Quand l'acquisition est terminée pour une position donnée de la source 10, l'unité de synchronisation 51 remet en marche les moteurs électriques 40 ou 140, pendant que les capteurs 13 se remettent à zéro. La mesure suivante peut ensuite être effectuée et mise en mémoire.

Il est encore possible de mouvoir en continu la source 10 et le détecteur 12. Le temps total d'exécution des mesures est alors sensiblement réduit car il n'y a plus d'arrêt des moteurs, mais il faut accepter un flou de l'image dû à la rotation au cours de chaque irradiation.

Il faut remarquer que les graduations 43, qui indiquent à l'unité de synchronisation 51 les positions des irradiations, ne sont pas nécessaires ; elles peuvent également être supprimées, et l'unité de synchronisation 51 détermine elle-même le cycle des irradiations par une horloge qui lui est incorporée.

Après l'acquisition de toutes les mesures, l'examen proprement dit de l'objet 11 est terminé. L'ensemble des informations passe dans l'unité de calcul 53 par une ligne 59 ; le calcul de la transformée de Radon ou de sa dérivée première est effectué, ainsi que leur inversion.

Les valeurs de la contribution locale à l'atténuation f(M) sont finalement acheminées par une ligne 60 vers les unités périphériques 54 qui contiennent en particulier des sorties graphiques et des écrans de visualisation.

Les opérations d'interpolation et de différentiation décrites ici ne doivent être considérées que comme des outils de calcul que d'autres peuvent remplacer sans sortir du cadre de l'invention ; de même les échantillonnages proposés ne sont que des exemples et peuvent varier dans des limites assez larges. Plusieurs des opérations de l'organigramme peuvent aussi être interverties.

Il faut cependant noter que l'expression de la dérivée de la transformée de Radon, la trajectoire circulaire et le faisceau conique imposent pratiquement, pour des raisons analytiques et de précision numérique, de travailler avec un réseau sphérique de points U, alors qu'il est naturel de représenter un objet, même de forme quasi-sphérique, à l'aide d'un réseau rectangulaire de points M dans lequel on peut définir des coupes planes. Plusieurs opérations d'interpolation sont donc indispensables pour passer d'un réseau à l'autre. En contrepartie, la densité du maillage des points M de l'objet 11 ne dépend pas rigoureusement du nombre de mesures défini par le nombre Nm : une certaine souplesse est donc possible à ce niveau.

Il est enfin évident que des dispositifs impliquant des mouvements de la source, de l'objet et du détecteur différents de ceux décrits ici à titre illustratif tombent encore dans le cadre de l'invention. De même, les mesures peuvent être effectuées plus rapidement par plusieurs sources fonctionnant simultanément et entre lesquelles les vues sont réparties.

L'invention fournit par conséquent un moyen intéressant d'obtenir des reconstructions tridimensionnelles à partir de mesures bidimensionnelles de l'atténuation d'un rayonnement. Une application plausible est évidemment l'imagerie médicale, une deuxième est le contrôle non destructif, mais tout objet 11 compatible avec les dimensions de l'appareillage et les rayonnements disponibles pourra être examiné. Les algorithmes utilisés calculent directement la solution, ce qui accélère les traitements par rapport notamment aux procédés par itérations. Enfin, le procédé garantit une localisation exacte des informations mesurées :

aucune distorsion de l'image n'apparaît, contrairement à ce qui se produit en utilisant d'autres procédés de l'art antérieur.

Suivant le dispositif de mesure et suivant les conventions utilisées, différents facteurs de normalisation ou changements d'échelles peuvent intervenir (étape 113 de l'organigramme). Les contributions locales à l'atténuation décrites dans le texte correspondent à des coefficients linéaires d'atténuation. Mais, on peut aussi exprimer, par exemple, les mesures d'atténuation en longueur d'eau équivalente, puis après reconstruction traduire le résultat des calculs en utilisant l'échelle d'Hounsfield, suivant les conventions pour les scanners X médicaux.

**Revendications**

**1.** Dispositif d'imagerie tridimensionnelle à partir de mesures bidimensionnelles de l'atténuation d'un rayonnement à travers un objet (11), comprenant une source (10) de rayonnement irradiant un espace de forme conique à partir d'un foyer (S), et dans lequel l'objet est placé, un détecteur (12) comprenant un dispositif bidimensionnel (13) mesurant l'atténuation du rayonnement à travers l'objet, un mécanisme (20, 30, 30') permettant d'effectuer une succession de mesures (11) sous des incidences différentes, ainsi qu'une chaîne de mesures (55) et une calculatrice (50) qui analysent et traitent les informations du dispositif bidimensionnel (13) lors des mesures pour en déduire la contribution locale f-(M) en différents points (M) d'un maillage de représentation de l'objet (11) à l'atténuation du rayonnement, caractérisé en ce que la source (10) est unique et en ce que la calculatrice (50) comprend des unités (53) programmées pour accomplir le calcul et l'inversion de la dérivée de la transformée de Radon de l'atténuation du rayonnement, la transformée de Radon d'une fonction étant définie comme l'ensemble des valeurs locales de cette fonction sur chaque plan passant par au moins un point du domaine sur lequel est définie la fonction, et la dérivée de la transformée de Radon étant définie comme la somme des taux de variation sur chacun desdits plans si on se déplace perpendiculairement audit plan dans le sens du vecteur normal défini par un système de coordonnées sphériques.

**2.** Dispositif d'imagerie tridimensionnelle selon la revendication 1, caractérisé en ce que la source (10) et le détecteur (12) restent à distance constante d'une origine fixe (O) pour les différentes incidences.

**3.** Dispositif d'imagerie tridimensionnelle selon la revendication 2, caractérisé en ce que le mécanisme comprend un rail circulaire (20) centré sur l'origine (O) qui guident la source (10) et le détecteur (12) pendant les mesures de telle façon qu'Ils restent toujours alignés avec l'origine O, à distance constante, la source décrivant une trajectoire circulaire Ce.

**4.** Dispositif d'imagerie tridimensionnelle selon la revendication 3, caractérisé en ce que le rail circulaire (20) est muni d'un pivot (21) permettant de déplacer la source selon deux trajectoires circulaires (Ce$_1$, Ce$_2$) décalées d'un angle constant ($\xi$).

**5.** Dispositif d'imagerie tridimensionnelle selon la revendication 3, caractérisé en ce que le rail circulaire (20) est muni d'un pivot (21) commandé par un moteur (22) commandé par la calculatrice (50) lui permettant de subir une rotation variable en fonction du temps.

**6.** Dispositif d'imagerie tridimensionnelle selon la revendication 2, caractérisé en ce que le mécanisme comprend deux rails circulaires (30, 30') parallèles, deux travées (31, 33) sur lesquelles coulissent respectivement la source (10) et le détecteur (12), ces travées (31, 33) étant placées toutes deux avec des positions opposées par rapport à l'origine (O) et parcourant les rails circulaires (30, 30') quand les mesures sont effectuées, les travées (31, 33) étant en outre recourbées en arc de cercle de manière à ce que leurs points soient à distance constante de l'origine (O), la source et le détecteur restant en permanence alignés et à distance constante de l'origine (O).

**7.** Dispositif d'imagerie tridimensionnelle selon l'une quelconque des revendications 3 à 6, caractérisé en ce que la source (10) et le détecteur (12) circulent sur les rails ou les travées (31, 33) à l'aide de moteurs électriques (40) pilotés par la calculatrice (50) à l'aide de dispositifs de repérage (41 et 43).

**8.** Dispositif d'imagerie tridimensionnelle selon la revendication 6, caractérisé en ce que les travées (31, 33) circulent sur les rails circulaires (30, 30') à l'aide de moteurs électriques pilotés par la calculatrice (50) à l'aide de dispositifs de repérage (41, 43).

9.  Dispositif d'imagerie tridimensionnelle selon la revendication 2, caractérisé en ce que la source (10) et le détecteur (12) sont reliés par une structure mécanique rigide (65, 75).

10. Dispositif d'imagerie tridimensionnelle selon la revendication 9, caractérisé en ce que la structure mécanique rigide (65) pivote autour d'un axe (67) passant par l'origine (O).

11. Dispositif d'imagerie tridimensionnelle selon la revendication 9, caractérisé en ce que la structure mécanique rigide (75) est immobile, et en ce qu'il comprend une autre structure mécanique (76 à 86) à laquelle l'objet (11) est fixé, e qui peut lui faire effectuer des rotations suivant au moins un axe passant par l'origine (O).

12. Procédé d'imagerie tridimensionnelle d'un objet (11) à partir de mesures bidimensionnelles de l'atténuation d'un rayonnement à travers l'objet par utilisation d'un dispositif formé d'une source (10) de rayonnement conique comprenant un foyer (S) et d'un détecteur bidimensionnel (12) formé d'un réseau de capteurs (13), caractérisé en ce qu'on calcule en chacun des premiers points (M) d'un maillage de représentation de l'objet (11) l'atténuation du rayonnement f(M) par calcul de grandeurs (R'f(U)) représentatives de la dérivée de la transformée de Radon de l'atténuation du rayonnement des points (U) d'un deuxième maillage de l'objet, la transformée de Radon d'une fonction étant définie comme l'ensemble des valeurs locales de cette fonction sur chaque plan passant par au moins un point du domaine sur lequel est définie la fonction, et la dérivée de la transformée de Radon étant définie comme la somme des taux de variation sur chacun desdits plans si on se déplace perpendiculairement audit plan dans le sens du vecteur normal défini par un système de coordonnées sphériques, les grandeurs (R'f(U)) étant calculées en effectuant la sommation pour chaque deuxième point (U), de la variation de l'atténuation du rayonnement le long d'au moins une ligne obtenue par intersection du détecteur (12) avec un plan passant par le foyer (S) du rayonnement conique et à proximité du deuxième point (U), la droite passant par l'origine (O) et le deuxième point (U) étant sensiblement orthogonale au plan passant par le foyer (S), puis des combinaisons linéaires de ces sommations, et l'atténuation du rayonnement en chacun des premiers points (M) étant obtenue par dérivation de ces grandeurs par rapport à la distance à l'origine (O), et enfin par combinaison linéaire (formule 6) des grandeurs dérivées, des interpolations étant effectuées par ailleurs pour passer des deuxièmes points (U) aux premiers points (M).

13. Procédé d'imagerie tridimensionnelle selon la revendication 12, caractérisé en ce que les grandeurs (Rf(U)) sont chacune déterminées par sommation de la variation de l'atténuation le long de lignes obtenues par l'intersection du détecteur (12) et de plans passant chacun par un point d'interpolation (U11, U12, U21, U22) proche du deuxième point (U) correspondant ainsi que par différentes positions du foyer (S) du rayonnement conique, la droite définie par l'origine (O) et chaque point d'interpolation (U11, U12, U21, U22) étant orthogonale au plan passant par ledit point d'interpolation.

14. Procédé d'imagerie tridimensionnelle selon l'une quelconque des revendications 12 ou 13, caractérisé en ce que les mesures sont effectuées quand le foyer (S) du rayonnement conique se trouve sur des plans méridiens distants d'angles réguliers ($2\pi$/Nm) et concourant en un axe passant par l'origine (O).

15. Procédé d'imagerie tridimensionnelle selon la revendication 14, caractérisé en ce que la source (10) et le détecteur (12) parcourent deux trajectoires (Tc, Tc') à distance constante de l'origine (O), sensiblement en forme de sinusoïde comprenant au moins deux périodes sur un tour complet autour de l'objet (11).

16. Procédé d'imagerie tridimensionnelle selon la revendication 14, caractérisé en ce que la source (10) et le détecteur (12) parcourent deux trajectoires (Tc, Tc') à distance constante de l'origine (O), sensiblement en forme de sinusoïde comprenant deux périodes sur un tour complet autour de l'objet (11) et dont l'amplitude (Int) est égale ou supérieure à la distance entre l'origine (O) et un point quelconque de l'objet (11), la distance entre les points de la trajectoire (Tc) et l'origine (O) étant par ailleurs égale ou supérieure à cette amplitude multipliée par $\sqrt{3}$.

17. Procédé d'imagerie tridimensionnelle selon l'une quelconque des revendications 14 à 16, caractérisé en ce que les deuxièmes points (U) pour lesquels on détermine les grandeurs R'f(U)) ont des coordonnées sphériques ($\rho$ (n), $\theta$(l), $\phi$(m)) régulièrement réparties et appartiennent aux plans perpendiculaires aux plans méridiens dans lesquels le foyer (S) du rayonnement conique se trouve lors des irradiations.

18. Procédé d'imagerie selon la revendication 17, caractérisé en ce que les grandeurs dérivées sont pondérées, interpolées et sommées à l'intérieur de chaque plan méridien pour obtenir des grandeurs Q($\phi$, B) en des troisièmes points (B) appartenant à un troisième maillage ayant des coordonnées rectangulaires (r(a), z(b)) régulièrement réparties sur des plans de projections réarrangées confondus avec les plans méridiens et appartenant aux mêmes plans orthogonaux aux plans méridiens que les points (M) du maillage de représentation de l'objet, et caractérisé en outre en ce que, pour tout point (B) du troisième maillage, les calculs de la quantité (Q ($\phi$, B)), caractéristique dudit point (B) sont regroupés avec ceux des autres points du troisième maillage appartenant au même plan de projections réarrangées.

19. Procédé d'imagerie tridimensionnelle selon la revendication 18, caractérisé en ce que l'atténuation f(M) en tout point (M) du maillage de représentation est obtenue par combinaison linéaire de quantités (Q($\phi$, B)) associées à des troisièmes points (B) de projections orthogonales des points (M) sur les plans de projections réarrangées.

**Claims**

1. Tridimensional imagery device from bidimensional measurements of the attenuation of radiation through an object (11), comprising a radiation source (10) irradiating a conical space from a focal spot (S) and in which the object is placed, a detector (12) comprising a bidimensional device (13) measuring the attenuation of the radiation through the object, a mechanism (20,30,30') making it possible to carry out a series of measurements (11) under different incidences, as well as a chain of measurements (55) and a computer (50) which analyse and process the information of the bidimensional device (13) at the time of the measurements so as to deduce from this the local contribution f(M) at different points (M) of a meshing representing the object (11) upon attenuation of radiation, characterized in that there is a single source (10) and in that the computer (50) comprises units (53) programmed for calculating and inverting the derivative of the Radon transform of the attenuation of the radiation, the Radon transform of a function being defined as all the local values of this function concerning each plane passing through at least one point of the range where the function is defined, and the derivative of the transformed Radon being defined as the sum of the variation rates on each of said planes if movement occurs perpendicular to said plane in the direction of the normal vector defined by a system of spherical coordinates.

2. Tridimensional imagery device according to claim 1, characterized in that the source (10) and detector (12) remain at a constant distance from a fixed origin (O) for the various incidences.

3. Tridimensional optical image formation device according to claim 2, characterized in that the mechanism comprises a circular rail (20) centered on the origin (O) which guides the source (10) and the detector (12) during the measurements so that they remain always aligned with the origin O at a constant distance, the source describing a circular trajectory Ce.

4. Tridimensional optical image formation device according to claim 3, characterized in that the circular rail (20) is provided with a pivot (21) enabling the source to be moved according to two circular trajectories (Ce1, Ce2) offset from a constant angle ($\xi$ ).

5. Tridimensional optical image formation device according to claim 3, characterized in that the circular rail (20) is provided with a pivot (21) controlled by a motor (22) controlled by the computer (50) enabling it to undergo a time-controlled variable rotation.

6. Tridimensional optical image formation device according to claim 2, characterized in that the mechanism comprises two parallel circular rails (30,30'), two sections (31,33) on which slide respectively are the source (10) and the detector (12), these sections (31,33) both being placed with opposing positions with respect to the origin (O) and traversing the circular rails (30,30') when the measurements are carried out, the sections (31,33) moreover being bent back as an arc of a circle so that their points are at a constant distance from the origin (O), the source and the detector remaining permanently aligned and at a constant distance from the origin (O).

7. Tridimensional optical image formation device according to any one of the claims 3 to 6, characterized in that the source (10) and the detector (12) move on the rails or sections (31,33) by means of electric motors (40) piloted by the computer (50) with the aid of marking devices (41 and 43).

8. Tridimensional optical image formation device according to claim 6, characterized in that the sections (31,33) move on the circular rails (30,30') by means of electric motors piloted by the computer (50) with the aid of marking devices (41,43).

9. Tridimensional optical image formation device according to claim 2, characterized in that the source (10) and the detector (12) are connected by a rigid mechanical structure (65,75).

10. Tridimensional optical image formation device according to claim 9, characterized in that the rigid mechanical structure (65) pivots around an axis (67) passing through the origin (O).

11. Tridimensional optical image formation device according to claim 9, characterized in that the rigid mechanical structure (75) is immobile and includes another mechanical structure (76 to 86) to which the object (11) is secured and which enables it to carry out rotations according to at least one axis passing through the origin (O).

12. Method for the tridimensional optical image formation of an object (11) from bidimensional measurements of the attenuation of a radiation through the object by using a device formed from a conical radiation source (10) comprising a focal spot (S) and a bidimensional detector (12) formed from a network of sensors (13), characterized in that in each of the first points (M) of a meshing representing the object (11), the attenuation of the radiation f(M) is calculated by calculating quantities (R'f(U)) representative of the derivative of the transformed Radon of attenuation of the radiation of the points (U) of a second meshing of the object, the transformed Radon of a function being defined as all the local values of this function concerning each plane passing through at least one point of the range where the function is defined, and the derivative of the transformed Radon being defined as the sum of the variation rates concerning each of said planes if movement occurs perpendicular to said plane in the direction of the normal vector defined by a system of spherical coordinates, the quantities (R'f(U)) being calculated by carrying out the summation for each second point (U) of the variation of attenuation of the radiation along at least one line obtained by the intersection of the detector (12) with a plane passing through the focal spot (S) of the conical radiation and in the proximity of the second point (U), the straight line passing through the origin (O) and the second point (U) being roughly orthogonal to the plane passing through the focal spot (S), then linear combinations of these summations, and the attenuations of the radiation at each of the first points (M) being obtained by derivation of these quantities with respect to the distance to the origin (O), and finally by linear combination (formula 6) of the derived quantities, interpolations being moreover carried out so as to pass from the second points (U) to the first points (M).

13. Tridimensional optical image formation method according to claim 12, characterized in that the quantities (Rf(U)) are each determined by summation of the variation of the attenuation along lines obtained by the intersection of the detector (12) and planes each passing through an interpolation point (U11,U12,U21,U22) close to the corresponding second point (U), as well as through different positions of the focal spot (S) of the conical radiation, the straight line defined by the origin (O) and each interpolation point (U11,U12,U21,U22) being orthogonal to the plane passing through said interpolation point.

14. Tridimensional optical image formation method according to either of claims 12 or 13, characterized in that the measurements are carried out when the focal spot (S) of the conical radiation occurs on meridian planes distant from regular angles ($2\pi$/Nm) and converging at an axis passing through the origin (O).

15. Tridimensional optical image formation method according to claim 14, characterized in that the source (10) and the detector (12) traverse two trajectories (Tc,Tc') at a constant distance from the origin (O) roughly in the shape of a sinusoid comprising at least two periods over a complete revolution around the object (11).

16. Tridimensional optical image formation method according to claim 14, characterized in that the source (10) and the detector (12) traverse two trajectories (Tc,Tc') at a constant distance from the origin (O), roughly in the form of a sinusoid comprising two periods over a complete revolution of the object (11) and whose amplitude (Int) is equal to or greater than the distance between the origin (O) and any point of the object (11), the distance between the points of the trajectory (Tc) and the origin (O) being moreover equal to or greater than this amplitude multiplied by $\sqrt{3}$.

17. Tridimensional optical image formation method according to any one of claims 14 to 16, characterized in that the second points (U) for which the quantities R'f(U) are determined have spherical coordinates $(\rho(n),\Theta(1),\phi(m))$ evenly distributed and belong to the planes perpendicular to the meridian planes in which the focal spot (S) of the conical radiation occurs at the time of irradiations.

18. Optical image formation method according to claim 17, characterized in that the derived quantities are weighted, interpolated and added up inside each meridian plane so as to obtain quantities Q $(\phi,B)$ at third points (B) belonging to a third meshing having rectangular coordinates (r(a), z(b)) evenly distributed over planes of rearranged projections of planes merged with the meridian planes and belonging to the same planes orthogonal to the meridian planes as the points (M) of the meshing representative of the object, and wherein also, for any point (B) of the third meshing, the calculations of the quantity (Q, $(\phi,B)$) characteristic of said point (B) are combined with those of the other points of the third meshing belonging to the same plane of rearranged projections.

19. Tridimensional optical image formation method according to claim 18, characterized in that the attenuation f(M) at any point (M) of the representative meshing is obtained by linear combination of the quantities (Q,$\phi$, B)) associated with the third points (B) of orthogonal projections of the points (M) on the planes with rearranged projections.

**Patentansprüche**

1. Vorrichtung zur dreidimensionalen Bilderzeugung aus zweidimensionalen Messungen der Schwächung einer Strahlung quer durch ein Objekt (11), umfassend eine Strahlungsquelle (10), die einen von einem Fokus (S) ausgehenden kegelförmigen Raumbereich bestrahlt, in dem sich das Objekt befindet, einen Detektor (12), der eine zweidimensionale Vorrichtung (13) umfaßt, die die Abschwächung der Strahlung quer durch das Objekt mißt, eine Mechanik (20, 30, 30'), die es erlaubt, eine Folge von Messungen (11) unter unterschiedlichem Einfall durchzuführen, sowie eine Meßkette (55) und einen Rechner (50), die bei den Messungen die Informationen der zweidimensionalen Vorrichtung (13) analysieren und verarbeiten, um daraus den lokalen Beitrag f(M) zur Abschwächung der Strahlung an verschiedenen Punkten (M) eines zur Darstellung des Objekts (11) dienenden Gitternetzes abzuleiten, dadurch gekennzeichnet, daß eine einzige Quelle (10) vorhanden ist und daß der Rechner (50) Einheiten (53) enthält, die programmiert sind, um die Berechnung und die Inversion der Ableitung der Radon-Transformierten der Abschwächung der Strahlung durchzuführen, wobei die RadonTransformierte einer Funktion definiert ist als die Gesamtheit der lokalen Werte dieser Funktion auf jeder beliebigen Ebene, die durch wenigstens einen Punkt des Bereichs geht, auf dem die Funktion erklärt ist, und die Ableitung der Radon-Transformierten definiert ist als die Summe der Änderungsraten auf jeder der genannten Ebenen, wenn man sich senkrecht zu genannter Ebene in Richtung des durch ein sphärisches Koordinatensystem definierten Normalenvektors bewegt.

2. Vorrichtung zur dreidimensionalen Bilderzeugung nach Anspruch 1, dadurch gekennzeichnet, daß für unterschiedlichen Einfall die Quelle (10) und der Detektor (12) in konstanter Entfernung zu einem festen Ursprung (O) bleiben.

3. Vorrichtung zur dreidimensionalen Bilderzeugung nach Anspruch 2, dadurch gekennzeichnet, daß die Mechanik eine im Ursprung (O) zentrierte kreisförmige Schiene (20) umfaßt, die die Quelle (10) und den Detektor (12) während der Messungen derart führt, daß sie in konstanter Entfernung vom Ursprung (O) immer mit diesem fluchten, wobei die Quelle eine kreisförmige Bahn (Ce) beschreibt.

4. Vorrichtung zur dreidimensionalen Bilderzeugung nach Anspruch 3, dadurch gekennzeichnet, daß die kreisförmige Schiene (20) mit einem Drehzapfen (21) versehen ist, der es erlaubt, die Quelle entsprechend zwei kreisförmigen Bahnen (Ce$_1$, Ce$_2$), die um einen konstanten Winkel ($\xi$) versetzt sind, zu bewegen.

5. Vorrichtung zur dreidimensionalen Bilderzeugung nach Anspruch 3, dadurch gekennzeichnet, daß die kreisförmige Schiene (20) mit einem Drehzapfen (21) versehen ist, die von einem Motor (22) gesteuert wird, der wiederum von dem Rechner (50) gesteuert wird, der es erlaubt, daß sie eine in Abhängigkeit von der Zeit veränderliche Rotation erfährt.

6. Vorrichtung zur dreidimensionalen Bilderzeugung nach Anspruch 2, dadurch gekennzeichnet, daß die Mechanik zwei kreisförmige parallele Schienen (30, 30') sowie zwei Bögen (31, 33) umfaßt, auf denen einerseits die Quelle (10), andererseits der Detektor (12) gleiten, wobei diese Bögen (31, 33) beide in in Bezug auf den Ursprung (O) entgegengesetzter Position angeordnet sind und auf den kreisförmigen Schienen (30, 30') laufen, wenn die Messungen durchgeführt werden, und ferner die Bögen (31, 33) kreisbogenförmig gekrümmt sind, damit ihre Punkte in konstanter Entfernung vom Ursprung (O) sind, während die Quelle und der Detektor andauernd fluchten und in konstanter Entfernung vom Ursprung (O) sind.

7. Vorrichtung zur dreidimensionalen Bilderzeugung nach einem der Ansprüche 3 bis 6, dadurch gekennzeichnet, daß die Quelle (10) und der Detektor (12) auf den Schienen oder den Bögen (31, 33) mit Hilfe von Elektromotoren (40) umlaufen, die von dem Rechner (50) mit Hilfe von Vorrichtungen zur Ortsbestimmung (41 und 43) gesteuert werden.

8. Vorrichtung zur dreidimensionalen Bilderzeugung nach Anspruch 6, dadurch gekennzeichnet, daß die Bögen (31, 33) auf den kreisförmigen Schienen (30, 30') mit Hilfe von Elektromotoren umlaufen, die von dem Rechner (50) mit Hilfe von Vorrichtungen zur Ortsbestimmung (41, 43) gesteuert werden.

9. Vorrichtung zur dreidimensionalen Bilderzeugung nach Anspruch 2, dadurch gekennzeichnet, daß die Quelle (10) und der Detektor (12) durch eine starre mechanische Struktur (65, 75) verbunden sind.

10. Vorrichtung zur dreidimensionalen Bilderzeugung nach Anspruch 9, dadurch gekennzeichnet, daß die starre mechanische Struktur (65) sich um eine Achse (67) dreht, die durch den Ursprung (O) geht.

11. Vorrichtung zur dreidimensionalen Bilderzeugung nach Anspruch 9, dadurch gekennzeichnet, daß die starre mechanische Struktur (75) unbeweglich ist und daß die Vorrichtung eine weitere mechanische Struktur (76 bis 86) umfaßt, an der das Objekt (11) befestigt ist und die das Objekt Rotationen um wenigstens eine durch den Ursprung (O) gehende Achse ausführen lassen kann.

12. Verfahren zur dreidimensionalen Bilderzeugung eines Objekts (11) aus zweidimensionalen Messungen der Abschwächung einer Strahlung quer durch das Objekt unter Verwendung einer Vorrichtung, die gebildet wird von einer Quelle (10) kegelförmiger Strahlung, umfassend einen Fokus (S) und einen aus einer Matrix von Empfängern (13) gebildeten zweidimensionalen Detektor (12), dadurch gekennzeichnet, daß man die Abschwächung der Strahlung f(M) in allen ersten Punkten (M) eines zur Darstellung des Objekts (11) dienenden Gitternetzes durch Berechnung der Größen (R'f(U)), die die Ableitung der Radon-Transformierten der Abschwächung des Strahlung in den Punkten (U) eines zweiten Gitternetzes des Objekts darstellen, berechnet, wobei die Radon-Transformierte einer Funktion definiert ist als die Gesamtheit der lokalen Werte dieser Funktion auf jeder Ebene, die durch wenigstens einen Punkt des Bereichs geht, auf dem die Funktion erklärt ist, und die Ableitung der Radon-Transformierten definiert ist als die Summe der Änderungsraten auf jeder der genannten Ebenen, wenn man sich senkrecht zu genannter Ebene in Richtung des durch ein sphärisches Koordinatensystem definierten Normalenvektors bewegt, wobei die Größen (R'f(U)) berechnet werden, indem man für alle zweiten Punkte (U) entlang wenigstens einer Linie, die erhalten wird durch Schnitt des Detektors (12) mit einer Ebene in der Nähe des zweiten Punktes (U), die durch den Fokus (S) der kegelförmigen Strahlung geht, wobei die durch den Ursprung (O) und den zweiten Punkt (U) gehende Gerade im wesentlichen orthogonal zu der Ebene ist, die durch den Fokus (S) geht, die Summen der Änderung der Abschwächung der

EP 0 292 402 B1

Strahlung und dann Linearkombinationen dieser Summen bildet, und wobei die Abschwächung der Strahlung in allen ersten Punkten (M) durch Berechnung der Ableitung dieser Größen nach der Entfernung zum Ursprung (O) und durch anschließende Linearkombination (Formel 6) der abgeleiteten Größen erhalten wird, wobei ferner Interpolationen durchgeführt werden, um von den zweiten Punkten (U) zu den ersten Punkten (M) zu gelangen.

13. Verfahren zur dreidimensionalen Bilderzeugung nach Anspruch 12, dadurch gekennzeichnet, daß die Größen (Rf(U)) alle bestimmt werden durch Summation der Änderung der Abschwächung entlang Linien, die erhalten werden durch den Schnitt des Detektors (12) mit Ebenen, die alle durch einen Interpolationspunkt (U11, U12, U21, U22) nahe dem entsprechenden zweiten Punkt (U) sowie durch unterschiedliche Positionen des Fokus (S) der kegelförmigen Strahlung gehen, wobei die Gerade, definiert durch den Ursprung (O) und jeden Interpolationspunkt (U11, U12, U21, U22), orthogonal zu der durch genannten Interpolationspunkt gehenden Ebene ist.

14. Verfahren zur dreidimensionalen Bilderzeugung nach einem der Ansprüche 12 oder 13, dadurch gekennzeichnet, daß die Messungen ausgeführt werden, wenn der Fokus (S) der kegelförmigen Strahlung sich auf Meridianebenen befindet, die durch regelmäßige Winkel ($2\pi/Nm$) getrennt sind und die in einer Achse, die durch den Ursprung (O) geht, zusammenlaufen.

15. Verfahren zur dreidimensionalen Bilderzeugung nach Anspruch 14, dadurch gekennzeichnet, daß in konstanter Entfernung vom Ursprung (O) die Quelle (10) und der Detektor (12) auf zwei Bahnen (Tc, Tc') im wesentlichen in Form einer Sinuskurve laufen, die wenigstens zwei Perioden für einen vollständigen Umlauf um das Objekt (11) umfaßt.

16. Verfahren zur dreidimensionalen Bilderzeugung nach Anspruch 14, dadurch gekennzeichnet, daß in konstanter Entfernung vom Ursprung (O) die Quelle (10) und der Detektor (12) auf zwei Bahnen (Tc, Tc') im wesentlichen in Form einer Sinuskurve laufen, die zwei Perioden für einen vollständigen Umlauf um das Objekt (11) umfaßt und deren Amplitude (Int) gleich oder größer als die Entfernung zwischen dem Ursprung (O) und einem beliebigen Punkt des Objekts (11) ist, wobei die Entfernung zwischen den Punkten der Bahn (Tc) und dem Ursprung (O) ferner gleich oder größer ist als diese Amplitude, multipliziert mit $\sqrt{3}$.

17. Verfahren zur dreidimensionalen Bilderzeugung nach einem der Ansprüche 14 bis 16, dadurch gekennzeichnet, daß die zweiten Punkte (U), für die man die Größen R'f(U) bestimmt, regelmäßig abgestufte sphärische Koordinaten ($\rho(n), \theta(1), \phi(m)$) haben und den zu den Meridianebenen senkrechten Ebenen angehören, in denen sich während der Bestrahlungen der Fokus (S) der kegelförmigen Strahlung befindet.

18. Verfahren zur dreidimensionalen Bilderzeugung nach Anspruch 17, dadurch gekennzeichnet, daß die abgeleiteten Größen gewichtet, interpoliert und im Inneren jeder Meridianebene summiert werden, um Größen Q($\phi$, B) in dritten Punkten (B) zu erhalten, die einem dritten Gitternetz angehören, das rechtwinklige Koordinaten (r(a), z(b)) hat, die regelmäßig auf Ebenen umgeordneter Projektionen verteilt sind, die mit den Meridianebenen zusammenfallen und denselben zu den Meridianebenen orthogonalen Ebenen angehören wie die Punkte (M) des zur Darstellung des Objekts dienenden Gitternetzes, und ferner dadurch gekennzeichnet, daß für jeden Punkt (B) des dritten Gitternetzes die Berechnungen der Größe (Q($\phi$,B)), charakteristisch für genannten Punkt (B), mit denen der anderen Punkte des dritten Gitternetzes, die derselben Ebene umgeordneter Projektionen angehören, zusammengefaßt werden.

19. Verfahren zur dreidimensionalen Bilderzeugung nach Anspruch 18, dadurch gekennzeichnet, daß die Abschwächung f(M) in jedem Punkt (M) des zur Darstellung dienenden Gitternetzes durch Linearkombination der Größen (Q($\phi$,B)) erhalten wird, die dritten Punkten (B) orthogonaler Projektionen von Punkten (M) auf die Ebenen umgeordneter Projektionen zugeordnet sind.

22

FIG. 1

FIG. 2

FIG. 3

FIG. 4

FIG. 5

26

FIG. 7

FIG. 6

27

FIG. 8

66

67

65

69

71

68

70

12

11

10

72

FIG 9

75

10

84

78

11

83

81

77

80

86

85

82

76

79

12

101 — MESURE DE L'ATTENUATION SUR LES CAPTEURS 13 : OBTENTION DE X(S,A) APRES CONVERSION LOGARITHMIQUE

102 — PONDERATION DE CORRECTION D'ELOIGNEMENT DU FOYER : $Y(S,A) = X(S,A).\dfrac{Rc}{SA}$

103 — FILTRAGES DANS LE PLAN DE DETECTION Pdet SUIVANT LES COORDONNEES p ET q : CALCUL DE $\dfrac{\partial Y}{\partial p}(S,A)$ ET $\dfrac{\partial Y}{\partial q}(S,A)$

104 — DEPLACEMENT DE LA SOURCE 10 ET NOUVELLE ACQUISITION

105 — SOMMATIONS SUR LES DROITES DM

106 — COMBINAISON LINEAIRE

107 — NORMALISATION : CALCUL DE R'f PAR LES FORMULES (4) ET (5)

108 — REARRANGEMENT : INTERPOLATIONS POUR CALCULER R'f(U)

109 — FILTRAGES : CALCUL DE $\dfrac{\partial R'f(U)}{\partial \rho}$

110 — PONDERATION PAR Sin θ

111 — RETROPROJECTIONS : CALCUL DE Q(φ,B)

112 — RETROPROJECTIONS : CALCUL DE f(M)

113 — NORMALISATION

VISUALISATION

FIG. 10